(19) Europäisches Patentamt
European Patent Office
Office européen des brevets

(11) Veröffentlichungsnummer: **0 185 987**
**A2**

(12) # EUROPÄISCHE PATENTANMELDUNG

(21) Anmeldenummer: 85115382.5

(22) Anmeldetag: 04.12.85

(51) Int. Cl.[4]: C 07 D 498/04
A 01 N 43/90
//(C07D498/04, 265:00, 249:00)

(30) Priorität: 20.12.84 JP 267443/84

(43) Veröffentlichungstag der Anmeldung:
02.07.86 Patentblatt 86/27

(84) Benannte Vertragsstaaten:
AT BE CH DE FR GB IT LI NL

(71) Anmelder: NIHON TOKUSHU NOYAKU SEIZO K.K.
No.4, 2-chome, Nihonbashi Honcho Chuo-ku
Tokyo 103(JP)

(72) Erfinder: Saito, Junichi
3-7-12, Osawa
Mitaka-shi Tokyo(JP)

(72) Erfinder: Kurahashi, Yoshio
47-15, Oya-machi
Hachioji-shi Tokyo(JP)

(72) Erfinder: Goto, Toshio
3454-21, Honmachida
Machida-shi Tokyo(JP)

(72) Erfinder: Yamaguchi, Naoko
4-6-7, Shinmei
Hino-shi Tokyo(JP)

(74) Vertreter: Schumacher, Günter, Dr. et al,
c/o Bayer AG Konzernverwaltung RP Patentabteilung
D-5090 Leverkusen 1 Bayerwerk(DE)

(54) **Triazolo-]3,2-c[perhydroxazin-8-on-Derivate.**

(57) Offenbart werden neue Triazolo-(3,2-c)perhydroxazin-8-on-Derivate der Formel (I)

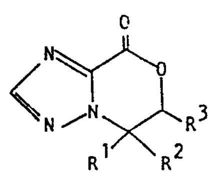

(I)

in welcher

$R^1$ ein Wasserstoff-Atom bezeichnet,

$R^2$ ein Wasserstoff-Atom, eine durch ein Halogen-Atom oder eine Phenyl-Gruppe substituierte Phenoxy-Gruppe oder eine durch ein Halogen-Atom substituierte Benzyl-Gruppe bezeichnet und

$R^1$ zusammen mit $R^2$ eine halogen-substituierte Benzyliden-Gruppe oder eine Cyclohexylmethyliden-Gruppe bilden kann und

$R^3$ eine tert-Butyl-Gruppe oder eine durch ein Halogen-Atom substituierte Phenyl-Gruppe bezeichnet, ein Verfahren zur Herstellung der neuen Stoffe und deren Verwendung als Pflanzenwachstumsregulatoren und Fungizide.

EP 0 185 987 A2

## Triazolo-(3,2-c)perhydroxazin-8-on-Derivate

Die vorliegende Erfindung betrifft neue Triazolo-(3,2-c)perhydroxazin-8-on-Derivate, ein Verfahren zu ihrer Herstellung sowie ihre Verwendung als Fungizide für Landwirtschaft und Gartenbau und als Pflanzen-Wachstumsregler.

Es wurde bereits offenbart, daß bestimmte Triazol-Derivate als Fungizide in der Landwirtschaft und im Gartenbau und/oder als Pflanzen-Wachstumsregler eingesetzt werden können (vgl. JP-OS 111 477/1980).

Nunmehr wurden neue Triazolo-(3,2-c)perhydroxazin-8-on-Derivate der Formel (I)

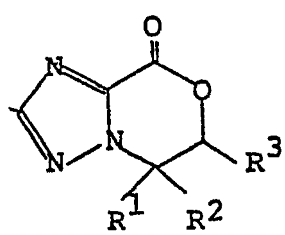

(I)

gefunden, in der

$R^1$ ein Wasserstoff-Atom bezeichnet,

$R^2$ ein Wasserstoff-Atom, eine durch ein Halogen-Atom oder eine Phenyl-Gruppe substituierte Phenoxy-Gruppe oder eine durch ein Halogen-Atom substituierte Benzyl-Gruppe bezeichnet und

$R^1$ zusammen mit $R^2$ eine halogen-substituierte Benzyliden-Gruppe oder eine Cyclohexylmethyliden-Gruppe bilden kann und

$R^3$ eine tert-Butyl-Gruppe oder eine durch ein Halogen-Atom substituierte Phenyl-Gruppe bezeichnet.

Triazolo-(3,2-c)perhydroxazin-8-on-Derivate der Formel (I) werden erhalten, wenn die Verbindungen der Formel (II)

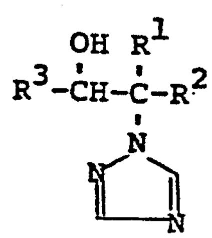

(II)

in der

$R^1$, $R^2$ und $R^3$ die im Vorstehenden angegebenen Bedeutungen haben, mit Trichlormethylchlorameisensäureester oder Phosgen, gegebenenfalls in Gegenwart von Säure-Acceptoren, umgesetzt werden.

Die neuen Triazolo-(3,2-c)perhydroxazin-8-on-Derivate zeigen hochwirksame, in Landwirtschaft und Gartenbau nutzbare fungizide Eigenschaften sowie auch das Pflanzenwachstum regulierende Eigenschaften.

Überraschenderweise zeigen die erfindungsgemäßen Triazolo-(3,2-c)perhydroxazin-8-on-Derivate beim Einsatz auf den Gebieten von Landwirtschaft und Gartenbau eine in beträchtlichem Maße herausragende fungizide Wirkung sowie ebenfalls überragende Wirksamkeit als Pflanzenwachstumsregler.

Unter den erfindungsgemäßen Triazolo-(3,2-c)perhydroxazin-8-on-Derivaten der Formel (I) sind diejenigen bevorzugt, in denen

$R^1$ ein Wasserstoff-Atom bezeichnet,

$R^2$ eine durch ein Halogen-Atom oder eine Phenyl-Gruppe substituierte Phenoxy-Gruppe oder eine durch ein Halogen-Atom substituierte Benzyl-Gruppe bezeichnet oder

$R^1$ zusammen mit $R^2$ eine halogen-substituierte Benzyliden-Gruppe oder eine Cyclohexylmethyliden-Gruppe bildet und

$R^3$ eine tert-Butyl-Gruppe bezeichnet.

Ganz besonders bevorzugte Triazolo-(3,2-c)perhydroxazin-8-on-Derivate der Formel (I) sind diejenigen, in denen

$R^1$ ein Wasserstoff-Atom bezeichnet,

$R^2$ eine 4-Chlorphenoxy-Gruppe, eine 4-Biphenyl-Gruppe oder eine 4-Chlorbenzyl-Gruppe bezeichnet oder

$R^1$ zusammen mit $R^2$ eine 4-Chlorbenzyliden-Gruppe oder eine Cyclohexylmethyliden-Gruppe bildet und

$R^3$ eine tert-Butyl-Gruppe bezeichnet.

Speziell erwähnt seien die folgenden Verbindungen:

Tabelle 1:

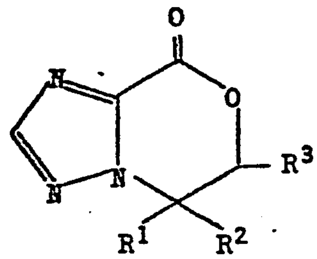 (I)

| $R^1$ | $R^2$ | $R^3$ |
| --- | --- | --- |
| H | 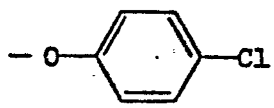 | tert-$C_4H_9$ |
| H | 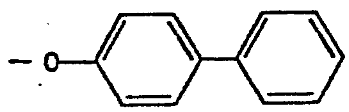 | tert-$C_4H_9$ |
| H | 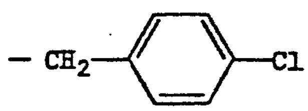 | tert-$C_4H_9$ |
| 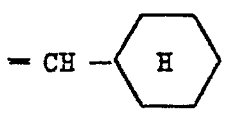 | | tert-$C_4H_9$ |
| 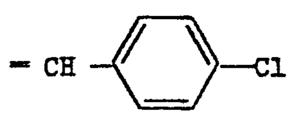 | | tert-$C_4H_9$ |

Wenn beispielsweise 1-Cyclohexyl-4,4-dimethyl-3-hydroxy-2-(1H-1,2,4-triazol-1-yl)pent-1-en und Trichlormethylchlorameisensäureester als Ausgangsstoffe eingesetzt werden, läßt sich die Umsetzung nach dem erfindungsgemäßen Verfahren durch die folgende Gleichung darstellen:

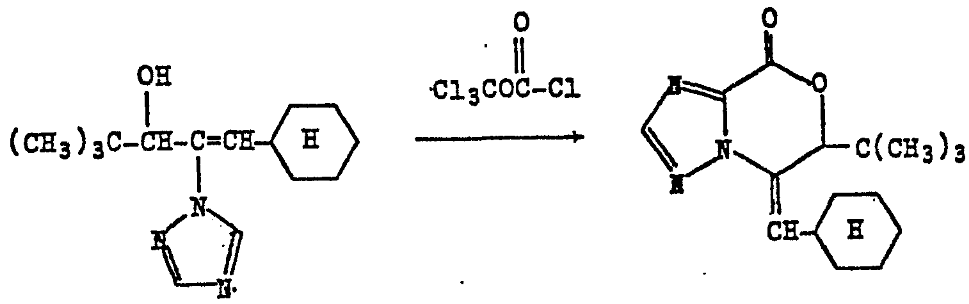


In der Formel (II) haben $R^1$, $R^2$ und $R^3$ die oben angegebenen Bedeutungen.

Bevorzugte Bedeutungen der Substituenten sind ebenfalls bereits im Vorstehenden aufgeführt.

Die gemäß der vorliegenden Erfindung einsetzbaren Verbindungen der Formel (II) sind bereits bekannt (vgl. die JP-OSen 111477/1980, 41875/1979, 28170/1978, 80738/1973, 13534/1975 und 23267/1976).

Als Beispiele seien erwähnt:
1-Cyclohexyl-4,4-dimethyl-3-hydroxy-2-(1H-1,2,4-triazol-1-yl)pent-1-en,
1-(4-Chlorphenoxy)-3,3-dimethyl-1-(1H-1,2,4-triazol-1-yl)butan-2-ol,
1-(4-Phenylphenoxy)-3,3-dimethyl-1-(1H-1,2,4-triazol-1-yl)butan-2-ol,

1-(4-Chlorphenyl)-4,4-dimethyl-2-(1H-1,2,4-triazol-1-yl)pentan-3-ol und
1-(4-Chlorphenyl)-4,4-dimethyl-3-hydroxy-2-(1H-1,2,4-triazol-1-yl)pent-1-en.

Bei dem erfindungsgemäßen Verfahren kann an Stelle von Trichlormethylchlorameisensäureester in gleicher Weise auch Phosgen als Reaktionskomponente eingesetzt werden.

Bei der Durchführung des erfindungsgemäßen Verfahrens können als Verdünnungsmittel sämtliche inerten organischen Lösungsmittel und auch Wasser eingesetzt werden. Vorzugsweise verwendbar sind: Wasser; aliphatische, cycloaliphatische und aromatische Kohlenwasserstoffe (die gegebenenfalls chloriert sein können), beispielsweise Hexan, Cyclohexan, Petrolether, Ligroin, Benzol, Toluol, Xylol, Methylenchlorid, Chloroform, Kohlenstofftetrachlorid, Ethylenchlorid, Trichlorethylen und Chlorbenzol; Ether, beispielsweise Diethylether, Methylethylether, Di-isopropylether, Dibutylether, Propylenoxid, Dioxan und Tetrahydrofuran; Ketone, beispielsweise Aceton, Methylethylketon, Methylisopropylketon und Methylisobutylketon; Nitrile, beispielsweise Acetonitril, Propionitril und Acrylnitril; Alkohole, beispielsweise Methanol, Ethanol, Isopropanol, Butanol und Ethylenglycol; Ester, beispielsweise Ethylacetat und Amylacetat; Säureamide, beispielsweise Dimethylformamid und Dimethylacetamid; Sulfone und Sulfoxide, beispielsweise Dimethylsulfoxid und Sulfolan; sowie Basen, beispielsweise Pyridin.

Das erfindungsgemäße Verfahren kann weiterhin in Gegenwart eines Säure-Acceptors durchgeführt werden. Als solche Säure-Acceptoren eingesetzt

werden können Hydroxide, Carbonate, Hydrogencarbonate und Alkoholate von Alkalimetallen, tertiäre Amine, beispielsweise Triethylamin, Diethylamin und Pyridin, die allgemein verwendet werden.

Die Reaktionstemperaturen können bei der Durchführung des erfindungsgemäßen Verfahrens innerhalb eines beträchtlichen Bereichs variiert werden. Im allgemeinen arbeitet man bei Temperaturen zwischen -20°C und dem Siedepunkt der Reaktionsmischung, vorzugsweise zwischen 0°C und dem Siedepunkt der Reaktionsmischung.

Die erfindungsgemäße Umsetzung kann unter dem Umgebungs-Druck durchgeführt werden, jedoch ist es auch möglich, unter erhöhtem oder vermindertem Druck zu arbeiten.

Bei der Durchführung des Verfahrens zur Herstellung der erfindungsgemäßen Verbindungen werden auf 1 mol der Verbindungen der Formel (II) etwa 0,5 bis 1,5 mol, vorzugsweise etwa 1 bis 1,2 mol, Trichlormethylchlorameisensäureester in einem inerten Lösungsmittel (Verdünnungsmittel) und, falls zweckmäßig, in Gegenwart von etwa 2 mol eines Säure-Acceptors (z.B. Pyridin) bei einer Temperatur von etwa 0°C bis etwa 5°C eingesetzt.

Im übrigen liegt die Reaktionstemperatur in dem Reaktionsschritt der Umsetzung des Trichloromethylchlorameisensäureesters vorzugsweise zwischen etwa 0°C und etwa 5°C, wobei nach Beendigung der Zugabe zur Vervollständigung der Reaktion auf Rückfluß-Temperatur erhitzt werden kann.

Die aktiven Substanzen gemäß der vorliegenden Erfindung zeigen stark ausgeprägte fungizide Effekte auf den Gebieten der Landwirtschaft und des Gartenbaus sowie Effekte der Regulierung des Pflanzenwachstums.

Dementsprechend können sie als fungizide Mittel in der Landwirtschaft und im Gartenbau sowie als Pflanzen-Wachstumsregler eingesetzt werden.

Fungizide Mittel werden im Pflanzenschutz eingesetzt zur Bekämpfung von

Plasmodiophoromycetes,
Oomycetes,
Chytridiomycetes,
Zygomycetes,
Ascomycetes,
Basidiomycetes und
Deuteromycetes,

beispielsweise von
Pulver-Mehltau (Sphaerotheca fuliginea) der Gurke,
Getreide-Mehltau (Erysiphe graminis), spez. der Gerste,
und
Blätterrost (Puccinia recondita).

Die aktiven Verbindungen gemäß der vorliegenden Erfindung greifen in den Stoffwechsel der Pflanzen ein und können aus diesem Grunde als Wachstumsregler eingesetzt werden.

Die bisherige Erfahrung hinsichtlich der Wirkungsweise von Pflanzen-Wachstumsreglern hat gezeigt, daß eine aktive Verbindung auch mehrere verschiedene Wirkungen auf Pflanzen ausüben kann. Die Wirkungen der Verbindungen hängen wesentlich von dem Zeitpunkt ihres Einsatzes, relativ zu dem Entwicklungsstadium der Pflanze,

sowie von den Mengen der auf die Pflanzen oder ihre Umgebung aufgebrachten aktiven Verbindungen und der Art, in der die Verbindungen zur Einwirkung gebracht werden, ab. In jedem Fall zielen die Pflanzen-Wachstumsregler auf eine Beeinflussung der Nutzpflanzen in einer ganz bestimmten gewünschten Weise.

Die das Pflanzenwachstum regulierenden Verbindungen können beispielsweise zur Hemmung des vegetativen Wachstums der Pflanzen eingesetzt werden. Eine solche Wachstumshemmung ist unter anderem von wirtschaftlichem Interesse im Falle von Gräsern, da es hierdurch möglich ist, die Häufigkeit des Mähens des Grases in Ziergärten, Parks und auf Sportplätzen, an Rändern und Rainen, auf Flugplätzen oder in Obstgärten zu vermindern. Auch die Hemmung des Wachstums von Grünpflanzen und Gehölzpflanzen an den Rändern und in der Nachbarschaft von Rohrleitungen oder Überlandleitungen oder, ganz allgemein, auf Flächen, auf denen ein starkes zusätzliches Pflanzenwachstum nicht erwünscht ist, ist von Bedeutung.

Die Verwendung von Wachstumsreglern zur Hemmung des Längenwachstums von Getreide ist ebenfalls bedeutsam. Die Gefahr, daß die Pflanzen sich vor der Ernte umlegen, wird dadurch verringert oder vollständig ausgeschaltet. Weiterhin vermögen Pflanzen-Wachstumsregler den Halm der Getreidepflanze zu festigen, was wiederum einem Umlegen entgegenwirkt. Der Einsatz von Wachstumsreglern zur Verkürzung und Festigung der Halme ermöglicht den Einsatz höherer Dünger-Mengen zur Steigerung des Ertrags ohne die Gefahr des Umlegens des Getreides.

Im Falle vieler Nutzpflanzen ermöglicht die Hemmung des vegetativen Wachstums eine dichtere Bepflanzung, so daß sich höhere Flächenerträge erzielen lassen. Ein Vorteil der auf diese Weise gewonnenen kleineren Pflanzen besteht auch darin, daß die Pflanzen leichter bearbeitet und geerntet werden können.

Die Hemmung des vegetativen Wachstums der Pflanzen kann auch zu einer Steigerung des Etrags führen, da die Nährstoffe und Assimilate der Blüten- und Fruchtbildung in höherem Maße zugute kommen als den vegetativen Teilen der Pflanze.

Auch eine Förderung des vegetativen Wachstums läßt sich häufig mit Hilfe von Wachstumsregulatoren erzielen. Dies ist dann von großem Nutzen, wenn es die vegetativen Pflanzenteile sind, die geerntet werden. Die Förderung des vegetativen Wachstums kann jedoch ebenfalls zu einem gleichzeitigen Wachstum des generativen Wachstums führen, da mehr Assimilate gebildet werden, so daß mehr Früchte oder größere Früchte erhalten werden.

Ertragssteigerungen lassen sich in manchen Fällen dadurch erreichen, daß der Pflanzen-Stoffwechsel beeinflußt wird, ohne daß erkennbare Veränderungen des vegetativen Wachstums stattfinden. Eine Änderung der Zusammensetzung der Pflanzen, die wiederum eine bessere Qualität der geernteten Produkte zur Folge haben kann, kann weiterhin mit Hilfe von Wachstumsreglern bewirkt werden. Auf diese Weise ist es beispielsweise möglich, den Zucker-Gehalt von Zuckerrüben, Zuckerrohr, Ananas oder Citrus-Früchten zu erhöhen oder den Protein-Gehalt in Sojabohnen oder Getreide zu erhöhen. Durch die Verwendung von Wachstumsreglern ist es beispielsweise auch

möglich, einen Abbau gewünschter Bestandteile, beispielsweise des Zuckers in Zuckerrüben oder Zuckerrohr, vor oder nach der Ernte zu hemmen. Es ist auch möglich, die Produktion oder das Ausströmen sekundärer Pflanzen-Bestandteile günstig zu beeinflussen. Die Stimulierung des Latex-Flusses in Gummibäumen sei als Beispiel hierfür erwähnt.

Parthenokarpe Früchte können unter dem Einfluß von Wachstumsreglern gebildet werden. Weiterhin läßt sich das Geschlecht der Blumen beeinflussen. Auch Sterilität von Pollen läßt sich erzeugen, was von großer Bedeutung bei der Züchtung und Gewinnung von Hybrid-Samen ist.

Die Verzweigung von Pflanzen läßt sich mit Hilfe von Wachstumsreglern steuern. Einerseits läßt sich durch Brechen der apicalen Dominanz die Entwicklung von Seitentrieben fördern, was sehr wünschenswert sein kann, insbesondere bei der Kultivierung von Zierpflanzen, auch in Verbindung mit einer Wachstumshemmung. Andererseits ist es jedoch auch möglich, das Wachsum von Seitentrieben zu hemmen. Es besteht großes Interesse an dieser Wirkung, beispielsweise bei der Kultivierung von Tabak oder beim Pflanzen von Tomaten.

Die Laubmenge von Pflanzen läßt sich unter dem Einfluß von Wachstumsreglern gezielt steuern, so daß eine Entlaubung der Pflanzen zu einem gewünschten Zeitpunkt bewirkt wird. Eine derartige Entlaubung ist von großer Bedeutung bei der mechanisch erfolgenden Ernte der Baumwolle; sie ist jedoch auch interessant für das Ernten anderer Nutzpflanzen, beispielsweise im Weinbau. Eine Entlaubung von Pflanzen kann auch zur Senkung der

Transpiration der Pflanzen vor dem Umpflanzen durchgeführt werden.

Das Abwerfen der Früchte bzw. Abfallen der Früchte läßt sich ebenfalls durch Wachstumsregler gezielt beeinflussen. Einerseits ist es möglich, daß vorzeitige Abfallen der Früchte zu verhindern. Auf der anderen Seite kann jedoch ein Abfallen der Früchte, oder sogar das Abfallen von Blüten, bis zu einem gewissen Grade (Ausdünnung) gefördert werden, um die Alternanz zu unterbrechen. Unter Alternanz versteht man die Eigenschaft mancher Obstarten, aus endogenen Gründen von Jahr zu Jahr sehr unterschiedliche Erträge zu liefern. Schließlich ist es mit Hilfe von Wachstumsreglern möglich, die zum Lösen der Frucht im Erntezeitpunkt erforderliche Kraft zu vermindern, um dadurch ein mechanisches Ernten zu ermöglichen oder ein manuelles Ernten zu erleichtern.

Weiterhin ist es auch möglich, durch den Einsatz von Wachstumsreglern den Reifeprozeß des Ernteprodukts vor oder nach dem Ernten zu beschleunigen oder zu verzögern. Dies ist von besonderem Vorteil, da hierdurch eine optimale Anpassung an die Bedürfnisse des Marktes ermöglicht wird. Weiterhin vermögen Wachstumsregler zuweilen die Färbung der Früchte zu verbessern. Zusätzlich ist es auch erreichbar, mit Hilfe von Wachstumsreglern das Reifwerden auf einen gewissen Zeitraum zu konzentrieren. Dies liefert die Voraussetzungen für die Möglichkeit, die Produkte vollständig mechanisch oder manuell in nur einem Arbeitsgang abzuernten, beispielsweise im Fall von Tabak, Tomaten und Kaffee.

Mit Hilfe von Wachstumsreglern ist es weiterhin möglich, die latente Periode der Samen oder Knospen von

Pflanzen zu beeinflussen, so daß die Pflanzen, wie beispielsweise Ananas oder Zierpflanzen in Baumschulen und Gärtnereien, zu einer Zeit keimen, treiben oder blühen, zu der sie normalerweise keine Bereitschaft dazu zeigen. Die Verzögerung des Treibens von Knospen oder der Keimung von Saatgut mit Hilfe von Wachstumsreglern kann in frostgefährdeten Gegenden wünschenswert sein, um Schäden durch späte Fröste zu vermeiden.

Schließlich vermögen Wachstumsregler auch die Resistenz von Pflanzen gegen Frost, Dürre oder hohen Salz-Gehalt im Boden zu induzieren. Hierdurch wird eine Kultivierung von Pflanzen in Gegenden ermöglicht, die gewöhnlich für diesen Zweck ungeeignet sind.

Die gute Tolerierung der aktiven Verbindungen durch die Pflanzen bei den zur Bekämpfung von Pflanzenkrankheiten erforderlichen Konzentrationen ermöglicht die Behandlung der oberirdischen Pflanzenteile, der Materialien zur vegetativen Vermehrung und des Saatguts sowie des Bodens.

Die aktiven Verbindungen können als gebräuchliche Formulierungen zubereitet werden, etwa als Lösungen, Emulsionen, Suspensionen, Pulver, Schäume, Pasten, Granulat, Aerosole, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chlorethylene oder Methylenchlorid, aliphatische Kohlenwasserstoffe wie Cyclohexan oder Paraffine, beispielsweise Erdöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist auch möglich, farbgebende Mittel, etwa anorganische Pigmente wie beispielsweise Eisenoxid, Titanoxid und Preußisch Blau oder organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der Erfindung können in den Formulierungen oder den verschiedenen Anwendungsformen im Gemisch mit anderen bekannten aktiven Verbindungen vorliegen, etwa mit Fungiziden, Bakteriziden, Insektiziden, Akariziden, Nematiziden, Herbiziden, vogelabweisenden Mitteln, Wachstumsregulatoren, Pflanzennährstoffen und Mitteln zur Verbesserung der Bodenstruktur.

Die aktiven Verbindungen können als solche oder in Form der aus ihnen durch weitere Verdünnung hergestellten Formulierungen oder verschiedenen Anwendungsformen zur Anwendung gebracht werden, etwa als gebrauchsfertige Lösungen, Emulsionen, Suspensionen, Pulver, Pasten und Granulate. Sie werden in der üblichen Weise ausgebracht, beispielsweise durch Bewässern, Eintauchen, Sprühen, Zerstäuben, Vernebeln, Verdampfen, Injizieren, Bilden einer Aufschlämmung, Aufpinseln, Stäuben, Streuen, Trockendüngung, Feuchtdüngung, Naßdüngung, Schlammdüngung oder Inkrustieren.

Im Falle der Verwendung der erfindungsgemäßen Stoffe als Fungizide in Landwirtschaft und Gartenbau können bei der Behandlung von Pflanzenteilen die Konzentrationen der aktiven Verbindungen innerhalb eines größeren Bereiches variiert werden. Im allgemeinen liegen die Konzentrationen zwischen 1 und 0,0001 Gew.-%, vorzugsweise zwischen 0,5 und 0,001 Gew.-%.

Bei der Saatgut-Behandlung werden allgemein Mengen der aktiven Verbindungen, bezogen auf die Saatgut-Menge, von 0,001 bis 50 g/kg, insbesondere von 0,01 bis 10 g/kg, eingesetzt.

Bei der Bodenbehandlung gelangen im allgemeinen Konzentrationen der aktiven Verbindungen am Wirkungsort von 0,00001 bis 0,1 Gew.-%, vorzugsweise von 0,0001 bis 0,02 Gew.-%, zur Anwendung.

Im Fall der Verwendung der erfindungsgemäßen Stoffe als Pflanzen-Wachstumsregler können die Aufwandmengen innerhalb eines beträchtlichen Bereichs variiert werden. Im allgemeinen werden Mengen der aktiven Verbindungen, bezogen auf die Oberfläche des Bodens, von 0,005 bis 10 kg/ha, vorzugsweise von 0,01 bis 5 kg/ha, eingesetzt.

Hinsichtlich der Einsatzzeit gilt die Regel, daß die Wachstumsregler innerhalb eines bevorzugten Zeitraums zur Anwendung gebracht werden, dessen genaue Festlegung von den klimatischen und den vegetativen Bedingungen abhängt.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Herstellungsbeispiele:

Beispiel 1

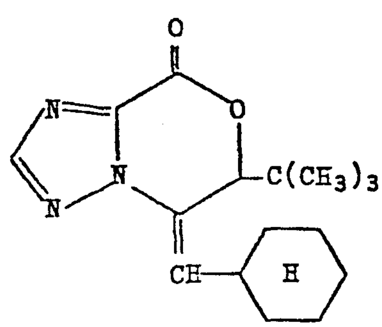

(Verbindung Nr. 1)

2,63 g 1-Cyclohexyl-4,4-dimethyl-3-hydroxy-2-(1H-1,2,4-triazol-1-yl)pent-1-en und 1,58 g Pyridin wurden in 30 ml Dichloromethan gelöst, und unter Eiskühlung wurden 1,98 g Trichlormethylchlorameisensäureester gelöst in 5 ml Dichlormethan tropfenweise hinzugefügt. Dann wurde nach einstündigem Rühren bei Raumtemperatur die Dichlormethan-Schicht mit Wasser gewaschen, getrocknet, und das Dichlormethan wurde unter vermindertem Druck abdestilliert, wonach 2,25 g des rohen gesuchten Produkts erhalten wurden. Im weiteren Verlauf wurden durch Reinigung mittels Säulen-chromatographie (Hexan : Ethylacetat = 2 : 1) 1,92 g des angestrebten 5-Cyclohexylmethyliden-6-tert.-butyl-(1,2,4)-triazolo-(3,2-c)perhydroxazin-8-ons erhalten. $n_D^{28}$ = 1,5264.

Die in gleicher Weise wie im vorstehenden Beispiel 1 hergestellten erfindungsgemäßen Verbindungen der Formel (I) sind in Tabelle 2 aufgeführt.

Tabelle 2

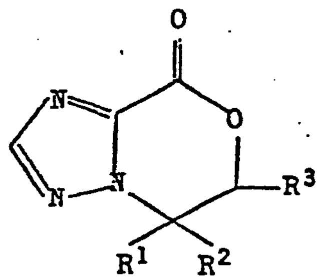

| Verbindung Nr. | $R^1$ | $R^2$ | $R^3$ | Physikal. Konstante |
|---|---|---|---|---|
| 2 | H | H | tert-$C_4H_9$ | Schmp. 143 - 150°C |
| 3 | H | H | 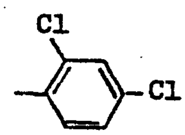 | Schmp. 172 - 185°C |
| 4 | H | 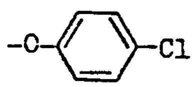 | tert-$C_4H_9$ | Schmp. 210 - 211°C |
| 5 | H | 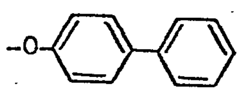 | tert-$C_4H_9$ | Öl |
| 6 | H | 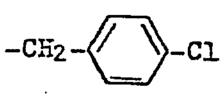 | tert-$€_4H_9$ | Schmp. 141 - 143°C |
| 7 | 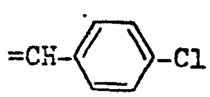 | | tert-$C_4H_9$ | Schmp. 140 - 141°C |

Verwendungsbeispiele:

Beispiel 2

Test auf Bekämpfung des Pulver-Mehltaus der Gurke:

Die Test-Verbindung in Form einer Emulsion wurde mit einer Sprühpistole auf in porösen Töpfen von 9 cm Durchmesser gezogene Gurken (Varietät: "Tokiwajibai) im 2-Blatt-Stadium aufgesprüht. Einen Tag nach dem Sprühen wurde eine Sporen-Suspension des Pathogens (Sphaerotheca fuliginea) durch Sprühen inokuliert. Nach Aufbewahrung der Töpfe in einem Raum mit einer konstanten Temperatur von 23°C wurde am zehnten Tage der Grad der Infektion aufgrund des nachfolgenden Bewertungsmaßstabs durch das Verhältnis der Läsionen zeigenden Flächen bestimmt, woraus der Bekämpfungseffekt berechnet wurde.

| Grad der Erkrankung | Fläche der Verletzungen (%) |
|---|---|
| 0 | 0 |
| 0,5 | 2 oder weniger |
| 1 | 3 bis 5 |
| 2 | 6 bis 15 |
| 3 | 16 bis 30 |
| 4 | 31 bis 50 |
| 5 | 51 oder mehr |

$$\text{Bekämpfungs-Wert (\%)} = \frac{\text{Grad der Infektion der unbehandelten Fläche} - \text{Grad der Infektion der behandelten Fläche}}{\text{Grad der Infektion der unbehandelten Fläche}} \times 100$$

0185987

Als Ergebnis zeigten die aktiven Verbindungen gemäß der vorliegenden Erfindung eine 100-prozentige Bekämpfungswirkung bei einer Wirkstoff-Konzentration von weniger als 50 ppm.

Beispiel 3

Test der Wachstumssteuerung von Reispflanzen:

Zubereitung der aktiven Verbindung (Emulsion):

| | |
|---|---|
| Aktive Verbindung: | 1 Gew.-Teil |
| Träger, Aceton: | 5 Gew.-Teile |
| Emulgator, Benzyloxypolyglycolether: | 1 Gew.-Teil |

Eine vorher festgelegte Menge der so formulierten Chemikalie wurde mit Wasser verdünnt, und die Verdünnung wurde für den Test verwendet.

Test-Verfahren

Feuchte Reisfeld-Erde wurde in eine Schale von 20 cm x 25 cm x 9 cm gefüllt. In diese Erde wurden Reis-Setzlinge im 2- bis 3-Blatt-Stadium (Varietät: Koshihikari) umgepflanzt, und dann wurde die Schale mit Wasser bis zu einer Tiefe von etwa 3 cm überflutet. Fünf Tage nach dem Umpflanzen wurde eine vorher festgelegte Menge der aktiven Verbindung mit Hilfe einer Pipette tropfenweise zur Behandlung der Wasseroberfläche zugegeben. Danach wurde die Überflutung mit etwa 3 cm Tiefe aufrechterhalten, und 20 Tage nach der chemischen Behandlung wurde die Höhe der Reispflanzen gemessen und zur Berechnung der Rate der Wachstumssteuerung mit derjenigen der Reispflanzen in unbehandelten Parzellen verglichen. Die Ergebnisse sind in Tabelle 3 aufgeführt.

Tabelle 3

| Verbindung Nr. | Aufwandmenge des wirksamen Bestandteils kg/ha | Wachstumshemmung in % |
|---|---|---|
| 1 | 2,0 | 66,3 |
| | 0,5 | 35,2 |
| | 0,25 | 23,1 |
| 6 | 2,0 | 73,2 |
| | 0,5 | 51,6 |
| | 0,25 | 37,8 |
| 7 | 2,0 | 78,4 |
| | 0,5 | 49,8 |
| | 0,25 | 46,4 |

0185987

## Patentansprüche

1. Triazolo-(3,2-c)perhydroxazin-8-on-Derivate der Formel (I)

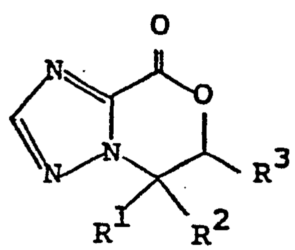

(I)

,

in der

$R^1$ ein Wasserstoff-Atom bezeichnet,

$R^2$ ein Wasserstoff-Atom, eine durch ein Halogen-Atom oder eine Phenyl-Gruppe substituierte Phenoxy-Gruppe oder eine durch ein Halogen-Atom substituierte Benzyl-Gruppe bezeichnet oder

$R^1$ zusammen mit $R^2$ eine halogen-substituierte Benzyliden-Gruppe oder eine Cyclohexylmethyliden-Gruppe bilden kann und

$R^3$ eine tert-Butyl-Gruppe oder eine durch ein Halogen-Atom substituierte Phenyl-Gruppe bezeichnet.

2. Verbindungen nach Anspruch 1, dadurch gekennzeichnet, daß
   - $R^1$ ein Wasserstoff-Atom bezeichnet,
   - $R^2$ eine durch ein Halogen-Atom oder eine Phenyl-Gruppe substituierte Phenoxy-Gruppe oder eine durch ein Halogen-Atom substituierte Benzyl-Gruppe bezeichnet oder
   - $R^1$ zusammen mit $R^2$ eine halogen-substituierte Benzyliden-Gruppe oder eine Cyclohexylmethyliden-Gruppe bildet und
   - $R^3$ eine tert-Butyl-Gruppe bezeichnet.

3. Verbindungen nach Anspruch 1, dadurch gekennzeichnet, daß
   - $R^1$ ein Wasserstoff-Atom bezeichnet,
   - $R^2$ eine 4-Chlorphenoxy-Gruppe, eine 4-Biphenyl-Gruppe oder eine 4-Chlorbenzyl-Gruppe bezeichnet oder
   - $R^1$ zusammen mit $R^2$ eine 4-Chlorbenzyliden-Gruppe oder eine Cyclohexylmethyliden-Gruppe bildet und
   - $R^3$ eine tert-Butyl-Gruppe bezeichnet.

4. Verbindungen nach Anspruch 1 bis 3, ausgewählt aus 5-(4-Chlorphenoxy)-6-tert.-butyl-(1,2,4)-triazolo-(3,2-c)perhydroxazin-8-on der Formel

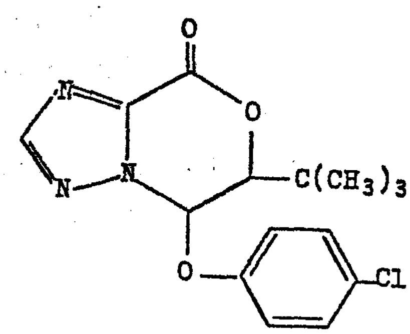

und

5-Cyclohexylmethyliden-6-tert.-butyl-(1,2,4)-triazolo-(3,2-c)perhydroxazin-8-on der Formel

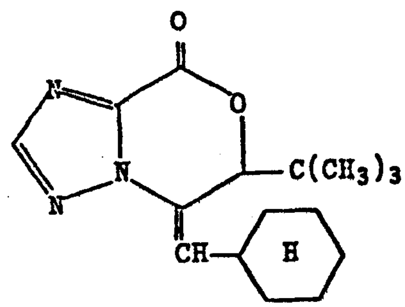

5. Verfahren zur Herstellung der Triazolo-(3,2-c)perhydroxazin-8-on-Derivate der Formel (I)

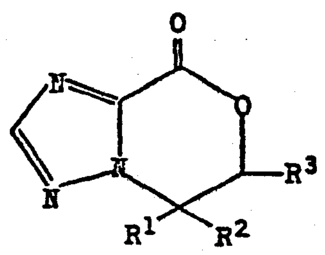

(I)

,

in der

$R^1$ ein Wasserstoff-Atom bezeichnet,

$R^2$ ein Wasserstoff-Atom, eine durch ein Halogen-Atom oder eine Phenyl-Gruppe substituierte Phenoxy-Gruppe oder eine durch ein Halogen-Atom substituierte Benzyl-Gruppe bezeichnet oder

$R^1$ zusammen mit $R^2$ eine halogen-substituierte Benzyliden-Gruppe oder eine Cyclohexylmethyliden-Gruppe bilden kann und

$R^3$ eine tert-Butyl-Gruppe oder eine durch ein Halogen-Atom substituierte Phenyl-Gruppe bezeichnet,

dadurch gekennzeichnet, daß Verbindungen der Formel (II)

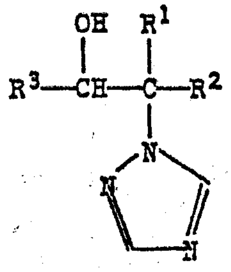

(II)

,

in der $R^1$, $R^2$ und $R^3$ die im Vorstehenden angegebenen Bedeutungen haben,

mit Trichlormethylchlorameisensäureester oder Phosgen umgesetzt werden.

6. Mittel zur Regulierung des Pflanzenwachstums oder fungizide Mittel, dadurch gekennzeichnet, daß sie wenigstens ein Triazolo-(3,2-c)-perhydroxazin-8-on-Derivat der Formel (I) enthalten.

7. Verfahren zur Regulierung des Pflanzenwachstums, dadurch gekennzeichnet, daß man Triazolo-(3,2-c)-perhydroxazin-8-on-Derivate der Formel (I) auf Pflanzen oder deren Lebensraum einwirken läßt.

8. Verfahren zur Bekämpfung von phytopathogenen Pilzen, dadurch gekennzeichnet, daß man Triazolo-(3,2-c)-perhydroxazin-8-on-Derivate der Formel (I) auf Pathogene und/oder den Ort ihres Auftretens und den Ort des Auftretens der phytopathogenen Pilze einwirken läßt.

9. Verwendung von Triazolo-(3,2-c)-perhydroxazin-8-on-Derivaten der Formel (I) zur Regulierung des Pflanzenwachstums oder zur Bekämpfung von phytopathogenen Pilzen.

Nit 188

10. Verfahren zur Herstellung von Mitteln zur Regulierung des Pflanzenwachstums oder von fungiziden Mitteln, dadurch gekennzeichnet, daß Triazolo-(3,2-c)-perhydroxazin-8-on-Derivate der Formel (I) mit Streckmitteln und/ oder oberflächenaktiven Mitteln vermischt werden.